# EUROPEAN PATENT APPLICATION

(11) **EP 1 547 474 A1**
(43) Date of publication of application: **29.06.2005**
(21) Application number: 03797612.3
(22) Date of filing: 16.09.2003
(51) Int. Cl.: A23L 1/30, A23F 3/16, A61K 31/353, A61K 35/78

(54) **FUNCTIONAL FOODS/DRINKS CONTAINING ANTIALLERGIC COMPONENT**

(30) Priority: 18.09.2002 JP 2002271730
(71) Applicant: Asahi Soft Drinks Co., Ltd., Sumada-ku, Tokyo 130-0001 (JP); MORINAGA & CO. LTD., Tokyo 108-0014 (JP); Incorporated Administrative Agency National Agriculture and Bio-oriented Research Organization, Ibaraki 305-0856 (JP); Kitani, Seiichi, Urayasu-shi, Chiba 279-0011 (JP)
(72) Inventor: NAGAI, Hiroshi, c/o Asahi Soft Drinks Co., Ltd, Moriya-shi, Ibaraki 302-0106 (JP); HASHIZUME, Shuichi, c/o Morinaga & Co., Ltd, Yokohama-shi, Kanagawa 230-8504 (JP); SATO, Susumu, c/o Morinaga & Co., Ltd, Yokohama-shi, Kanagawa 230-8504 (JP); YAMAMOTO, Mari, Kakegawa-shi, Shizuoka 436-0069 (JP); KITANI, Seiichi, Urayasu-shi, Chiba 279-0011 (JP)
(74) Representative: Merkle, Gebhard
(86) International application number: PCT/JP2003/011775
(87) International publication number: WO 2004/026047

(57) **Abstract**

The invention relates to functional food/drink with antiallergic actions, which contain one or more selected from the group consisting of epigallocatechin-3-O-(3-O-methyl)gallate and gallocatechin-3-O-(3-O-methyl)gallate, epigallocatechin-4-O-(4-O-methyl)gallate and gallocatechin-4-O-(4-O-methyl)gallate, and strychnine.

## Description

### FIELD OF THE INVENTION

The present invention relates to a functional food/drink containing an effective amount of a tea extract ingredient with an antiallergic action.

### BACKGROUND OF THE INVENTION

Men and women of today individually pay attention to health and actively take healthy functional foods for the purpose of their health control, so as to find a way out of deadlocks of environmental pollution and in medical systems. A great number of reports tell the efficacy of tea (green tea, fermented tea) in particular. Tea is a healthy functional food and drink most widely utilized traditionally, Tea has been known to have for example antioxidative action, antimutagenicity, antibacterial action, antiviral action, an action suppressing the increase of blood glucose, and hypotensive action.

However, no products for the purpose of giving such definite actions have been provided. Currently, tea is taken on a simple image that tea drinks or foods containing tea are healthy. Additionally, a broad sense of tea including for example Guava tea and Tencha except genuine tea (Camellia Sinensis) also exists, along with tea produced by specific processing methods such as Gavaron tea. Some of them have been approved of their specific examined functions.

Meanwhile, a material from which an ingredient with an anti-allergic action can be readily ingested with safety is demanded by a great number of people afflicted with allergic diseases such as pollinosis, who are said to be a total of 13,000,000. The demand is very high from potential such patients never visiting medical facilities, so the demand from market is also extremely high.

### SUMMARY OF THE INVENTION

In such circumstances, the invention has been achieved. The object is to provide functional food and drinks safely ingestible with an anti-allergic action for patients afflicted with allergic diseases such as pollinosis and common consumers.

With attention focused on water-soluble anti-allergic ingredients contained in some green tea, namely EGCG3"Me, GCG3"Me, EGCG4"Me, GCG4"Me and strychnine so as to achieve the object described above, in accordance with the invention, foods and drinks containing at least one of these ingredients are obtained.

More specifically, the invention provides those described below.
(1) A functional food/drink comprising one or more selected from the group consisting of epigallocatechin-3-O-(3-O-methyl)gallate (referred to as EGCG3"Me hereinafter) and gallocatechin-3-O-(3-O-methyl)gallate (referred to as GCG3"Me hereinafter) as an optical isomer thereof, epigallocatechin-4-O-(4-O-methyl)gallate (referred to as EGCG4"Me hereinafter) and gallocatechin-4-O-(4-O-methyl)gallate (referred to as GCG4"Me hereinafter) as an optical isomer thereof, and strychnine.
(2) The functional food/drink according to (1), wherein EGCG3"Me, GCG3"Me, EGCG4"Me, GCG4"Me and strychnine are obtained as tea extracts and/or are contained in ground tea.
   In other words, the functional foods and drinks in accordance with the invention may satisfactorily be tea extracts themselves (namely, so-called common "tea") or may contain tea extracts or may contain ground tea (including tea leaf, tea stem and all other things similar to them) therein (for example, powdered green tea, purine of powdered green tea, and ice cream of powdered green tea).
(3) The functional food/drink according to (2), wherein the tea is of Assam hybrid and/or Chinese species.
   As described below, the Assam hybrid Includes for example "Benifuki", "Benihomare", and "Benifuji". The Chinese species includes for example "Seishin dai-pan", "Selshin oolong", and "Oba oolong".
(4) The functional food/drink according to (3), wherein the Assam hybrid is one or more selected from the group consisting of "Benifuki", "Benihomare", and "Benifuji" and the Chinese species is one or more selected from the group consisting of "Seishin dai-pan", "Seishin oolong", and "Oba oolong".
(5) The functional food/drink according to any one of (1) to (4), wherein one or more ingredients of EGCG3"Me, GCG3"Me, EGCG4"Me, and GCG4"Me are contained at 0.01 mg to 1,000 mg per one liter to daily intake of 0.3 mg to 3,000 mg.
   Herein, the daily intake can be calculated by integrating the concentration and the volume. More specifically, the daily intake can be adjusted by adjusting the concentration and container volume. For the dally intake of 0.3 mg, for example, such drink at a concentration of 1 mg/one liter is placed in a 300-ml container.
(6) The functional food/drink according to any one of (1) to (4), wherein one or more ingredients of EGCG3"Me, GCG3"Me, EGCG4"Me, and GCG4"Me are contained at 1 mg to 500 mg per one liter to daily intake of 3 mg to 300 mg.
(7) The functional food/drink according to any one of (1) to (6), wherein strychnine is contained at 0.01 mg to 1,000 mg per one liter to daily intake of 0.3 mg to 3,000 mg.
(8) The functional food/drink according to any one of (1) to (6), wherein strychnine is contained at 1 mg to 500 mg per one liter to daily intake of 3 mg to 300 mg,
(9) The functional food/drink according to any one of (1) to (8), further comprising a masking agent to mask bitterness.
(10) A tea bag sealed tea comprising one or more selected from the group consisting of EGCG3"Me, GCG3"Me, EGCG4"Me, GCG4"Me and strychnine therein.

### BRIEF DESCRIPTION OF THE DRAWINGS

Fig. 1 shows bar graphs of contents of ingredients vs. extraction temperature.

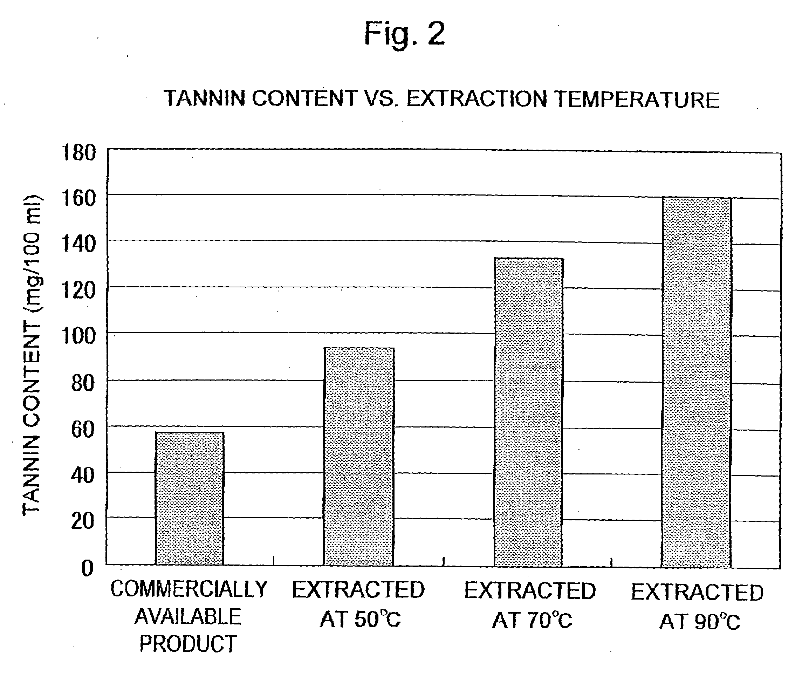

Fig. 2 shows bar graphs of tannin content vs. extraction temperature.

### DETAILED DESCRIPTION OF THE PREFERRED EMBODIMENT

One or more ingredients of EGCG3"Me, GCG3"Me, EGCG4"Me, and GCG4"Me (these substances are collectively referred to as "methylcatechin" hereinafter) and strychnine can be extracted as water-soluble ingredients from tea, _particularly the Assam hybrid mainly for black tea, including for example "Benifuki", "Benihomare", and "Benifuji" and the Chinese species including for example "Seishin dai-pan", "Seishin oolong", and "Oba oolong".

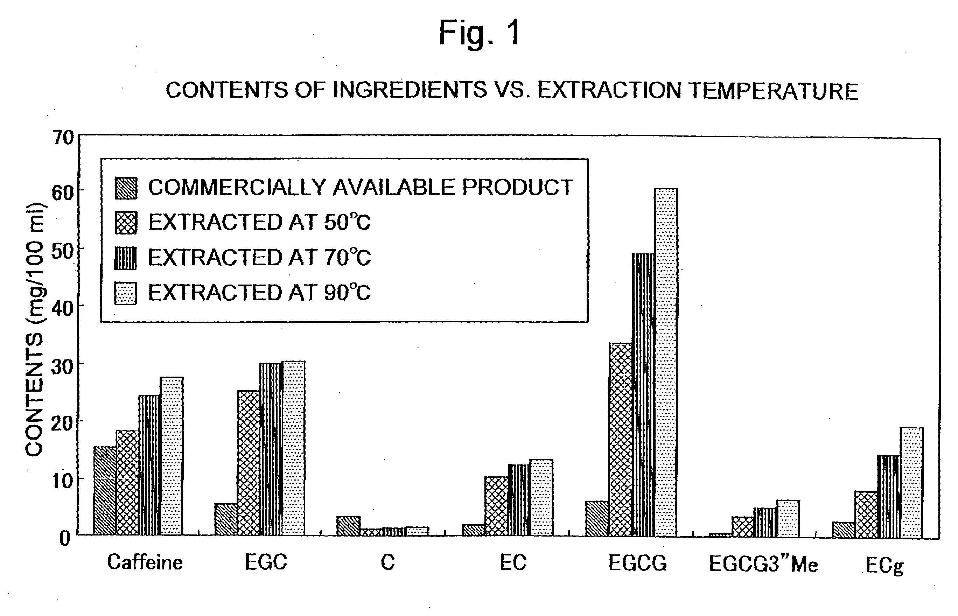

The extracted amount thereof has a correlation with the temperature of water for use in the extraction. As the water temperature is higher in the order of 50°C, 70°C and 90°C, the extracted amount is larger (Fig.1). In Fig.1, EGCG3"Me is methylcatechin. In other words, the contents of such ingredients can be adjusted by controlling the temperature for use in the extraction. The contents can be assayed by HPLC.

Then, the solvent includes for example but is not limited to water and solvents usable in foods such as alcohol at high extraction efficiency thereof. Additionally, the performance of strychnine follows methylcatechin.

The extraction temperature may be appropriate but is preferably a high temperature within a range under consideration of flavor balance with umami and the like extractable at low temperature, because the active ingredients are extracted at high temperature.

Extract solutions containing the active ingredients can generally be powdered by routine spray drying, freeze-drying and the like. Methylcatechin was recovered at 80% or more by drying with spray dryer, where the content was 2 to 3%. The performance of strychnine follows methylcatechin.

The tannin content therein is higher than those in existing green tea drinks. If necessary, however, juices of citrus species such as grape fruit, bitterness-masking peptides and the like may be used as ingredients for masking bitterness.

Concerning the anti-allergic actions of methylcatechin and strychnine, herein, it is suggested that strychnine suppresses IgE generation at an early reaction stage, while methyicatechin inhibits the activation of mast cell.

So as to get the anti-allergic action in a clinical sense, the effective amount of methylcatechin is 0.01 mg to 1,000 mg per one liter. Preferably, the effective amount is 1 mg to 50 mg per one liter. Preferably, adults may take 200 ml of the resulting drink per one intake, three times or more dally.

Because methylcatechin is a water-soluble ingredient, an excess of methylcatechin is immediately excreted outside even when a large amount thereof is ingested.

The active amount of strychnine is 0.01 mg to 1,000 mg, Preferably, the active amount is 1 mg to 50 mg. Strychnine is also a water-soluble ingredient. Accordingly, an excess of strychnine is immediately excreted outside.

### [Example 1]

"Benifuki" primary tea (picked on May 2; opening at 34%; one-hour drying in the sun for shrinking; provided by Yasai Cha-gyo Kenkyusho (Vegetable-Tea Industry Research Institute) of 30 g as a raw material and pure water of a 30-fold volume were used for extraction at three levels of temperature (50, 70, 90°C). The resulting extract solutions were adjusted with pure water to a volume of one liter. These tea extract solutions were subjected to tests.

Tests were done by carrying out the fundamental analysis of methylcatechin contained in the tea extract solutions and evaluating the results. Caffeine and catechins were analyzed by HPLC; and tannin analysis was done by iron tartrate method. Catechin standards were purchased from Funakoshi Co., Ltd. and EGCG3"Me was purchased from Nakahara Science Co., Ltd.

The intended EGCG3"Me was readily separated and assayed by general methods. Additionally, the content thereof depended on the extraction temperature. As the extraction temperature was higher, the extracted amount increased at a higher concentration (Fig.1). This was also observed about other catechins.

After getting the approval of the Institutional Review Board of the Medical Department, a clinical test was carried out from the clinical standpoint by the internal Medicine Department of allergy rheumatism, the Affiliated Hospital of Tokyo University. The intake of EGCG3"Me was estimated to be 10 to 45 mg daily.

The results of the test indicate that 15 mg methylcatechin could be ingested by drinking about 230 ml of an extract of 10 g of tea leaves per one liter at 90°C, about 290 ml of an extract of 10 g of tea leaves per liter at 70°C and about 450 ml of an extract of 10 g of tea leaves per liter at 50°C.

### [Example 2]

Using a powder of an extract of "Benifuki", a hard candy was prepared. Methylcatechin was contained at 2.7% in the powder of the extract, so one hard candy was designed to contain 3 mg of methylcatechin. Consequently, methylcatechin was recovered at a yield of 104%, without any methylcatechin modification during the process of preparing the hard candy. Strychnine was also stable. Additionally, the hard candy was delicious and ingestible every day with almost no bitterness.

### [Example 3]

One or two packs (daily) of a teabag containing 2 g of "Benifuki" were given to 46 allergic patients. Three months later, the improvement of allergic symptoms such as cough, nasal congestion and itching was observed in 26 cases (78%). At a test 6 months later, apparent decrease of IgE-RIST was observed in 5 cases (23%) and apparent decrease of eosinophil counts was confirmed in 9 cases (43%). Accordingly, an effect on the improvement was observed. Additionally, no adverse effects were observed.

### [Example 4]

10 g each of "Benifuki", "Benifuji" and "Benihomare" as raw materials and pure water of a 30-fold volume were used for extraction at 90°C. The resulting extract solutions were adjusted with pure water to a volume of 3 L, to which adjusters of water quality such as sodium bicarbonate and vitamin C were added for mixing them together. Further, the mixtures were divided into two portions. "Benifuki" ground to about mean particle size of 8 µm was mixed into one of the portions under agitation. Each of the mixture solutions was sterilized and sealed in nitrogen in a sealed container.

The taste of the resulting two types of product solutions was evaluated by 10 taste panelists. Seven among the 10 panelists liked the products with addition of "Benifuki" and highly evaluated the products as genuine tea.

Because the extracted amounts of catechin and strychnine vary depending on the various conditions such as extraction solvent and extraction temperature as described above and additionally because tea itself contains variable amounts of catechin and strychnine, such extract solutions at higher concentrations may satisfactorily be diluted to a concentration suitable for drinking, with no specific problem.

As described above, it was confirmed that tea containing methylcatechin and strychnine with anti-allergic actions, particularly extracts of "Benifuki", "Benihomare" and "Benifuji", when ingested, could exert the anti-allergic actions in bodies.

As described above, the foods are effective for primary prophylaxis of allergy in general consumers when ingested regularly and for secondary prophylaxis in patients afflicted with allergic diseases. Additionally, it is considered that such foods can reduce the amounts of pharmaceutical agents such as steroid under appropriate instructions of medical doctors, leading to the saving of increasing medical expenses.

## Claims

1. A functional food/drink comprising one or more selected from the group consisting of epigallocatechin-3-O-(3-O-methyl)gallate (referred to as EGCG3"Me hereinafter) and gallocatechin-3-O-(3-O-methyl)gallate (referred to as GCG3"Me hereinafter) as an optical isomer thereof, epigallocatechin-4-O-(4-O-methyl)gallate (referred to as EGCG4"Me hereinafter) and gallocatechin-4-O-(4-O-methyl)gallate (referred to as GCG4"Me hereinafter) as an optical isomer thereof, and strychnine.

2. The functional food/drink according to claim 1, wherein EGCG3"Me, GCG3"Me, EGCG4"Me, GCG4"Me and strychnine are obtained as tea extracts and/or are contained in ground tea.

3. The functional food/drink according to claim 2, wherein the tea Is of Assam hybrid and/or Chinese species.

4. The functional food/drink according to claim 3, wherein the Assam hybrid is one or more selected from the group consisting of "Benlfuki", "Benihomare", and "Benifuji" and the Chinese species is one or more selected from the group consisting of "Seishin dai-pan", "Seishin oolong", and "Oba oolong".

5. The functional food/drink according to any one of claims 1 to 4, wherein one or more ingredients of EGCG3"Me, GCG3"Me, EGCG4"Me, and GCG4"Me are contained at 0.01 mg to 1,000 mg per one liter to daily intake of 0.3 mg to 3,000 mg.

6. The functional food/drink according to any one of claims 1 to 4, wherein one or more ingredients of EGCG3"Me, GCG3"Me, EGCG4"Me, and GCG4"Me are contained at 1 mg to 500 mg per one liter to daily intake of 3 mg to 300 mg.

7. The functional food/drink according to any one of claims 1 to 6, wherein strychnine is contained at 0.01 mg to 1,000 mg per one liter to daily intake of 0.3 mg to 3,000 mg.

8. The functional food/drink according to any one of claims 1 to 6, wherein strychnine is contained at 1 mg to 500 mg per one liter to daily intake of 3 mg to 300 mg.

9. The functional food/drink according to any one of claims 1 to 8, further comprising a masking agent to mask bitterness.

10. A tea bag sealed tea comprising one or more selected from the group consisting of EGCG3"Me, GCG3"Me, EGCG4"Me, GCG4"Me and strychnine therein.
